# EUROPÄISCHE PATENTSCHRIFT

(11) **EP 0 966 984 B1**
(45) Veröffentlichungstag und Bekanntmachung des Hinweises auf die Patenterteilung: **17.08.2005**
(21) Anmeldenummer: 99111745.8
(22) Anmeldetag: 17.06.1999
(51) Int. Cl.: A61M 39/04, A61M 39/14

(54) **Konnektorelement**
Connector element
Elément connecteur

(30) Priorität: 26.06.1998 DE 19828650
(43) Veröffentlichungstag der Anmeldung: 29.12.1999
(73) Patentinhaber: Fresenius Medical Care Deutschland GmbH, 61352 Bad Homburg v.d.H. (DE)
(72) Erfinder: Lauer, Martin, 66606 St. Wendel (DE)
(74) Vertreter: Gossel, Hans K., Dipl.-Ing.

(56) Entgegenhaltungen:
- EP-A- 0 830 874
- US-A- 4 161 949
- US-A- 4 195 632
- US-A- 4 457 749
- US-A- 4 636 204

## Beschreibung

Die vorliegende Erfindung betrifft ein Konnektorelement insbesondere zum Verbinden von Schläuchen, Kanülen und Kathetern mit einem Bereich zur Führung eines durchströmenden Mediums, mit einem Absperrelement, durch das der Bereich zur Führung des Mediums verschließbar ist sowie mit einem Bereich zur Aufnahme eines Durchstoßkörpers, der derart ausgestaltet ist, daß der Durchstoßkörper relativ zu dem Absperrelement bewegbar ist und das Absperrelement bei der Konnektion durch den Durchstoßkörper geöffnet wird.

Ein wesentliches Verwendungsgebiet für Konnektoren ist die Verbindung eines Behälters über eine Schlauchleitung mit einem medizinischen Arbeitsmittel, beispielsweise mit einem Dialysator, oder die Verbindung zweier Schlauchenden zum Zwecke der Bereitstellung von Verlängerungsleitungen. Um stets eine sterile und dichte Verbindung sicherzustellen und somit eine Gefährdung von Patienten sicher auszuschließen, werden hohe Anforderungen an die Ausführung und Qualität der Konnektoren sowie an die Art der Verbindungsbildung der Konnektoren gestellt, wodurch eine Kontamination nicht nur vor, sondern auch während und nach der Konnektion verhindert werden soll.

In der WO 82/02528 wird ein aus zwei Konnektorelementen aufgebautes Konnektionssystem offenbart, bei dem die Konnektorelemente vor der Konnektion mittels thermoplastischer Membranen verschlossen sind. Die Konnektorelemente sind an ihrem von der thermoplastischen Membran abgewandten Ende jeweils mit einem Schlauch verbunden, der seinerseits beispielsweise mit einem Behälter zur Aufnahme von Dialysierflüssigkeiten oder mit einem Dialysegerät in Verbindung steht. Die Konnektorelemente weisen zur Öffnung von in den Schläuchen angeordneten Schlauchmembranen ein spitz zulaufendes Ende auf. Bei der Konnektion werden zunächst die thermoplastischen Membranen in Verbindung gebracht und mittels einer geeigneten Wärmequelle geschmolzen, wodurch eine dichte Verbindung der Konnektorelemente entsteht. Im Anschluß daran erfolgt die Öffnung der Schlauchmembranen mittels der spitzen Enden der Konnektorelemente, wobei die Bewegung der spitzen Enden relativ zu den Schlauchmembranen durch einen balgartig ausgeführten Bereich des Schlauches ermöglicht wird. Der Konnektor stellt ein komplex aufgebautes System aus unterschiedlichen Membranen und Konnektorelementen auf, was die Herstellung der Konnektoren entsprechend aufwendig und teuer gestaltet.

Die WO 94/08173 beschreibt ein Konnektorsystem mit zwei Konnektorelementen, die jeweils durch Membranen verschlossen sind. Nach dem Zusammenführen der Membranen wird ein verschiebbarer Dorn durch die Membranen geführt, der in einem der Konnektorelemente bewegbar angeordnet ist. Im nicht konnektierten Zustand ist der Dorn in einem Raum aufgenommen, der von einer der Membranen begrenzt wird. Entsprechend ist es bei der Herstellung erforderlich, die Membran nach Einführung des Dorns nachträglich am Konnektorelement zu fixieren, was den Herstellvorgang des Konnektors verhältnismäßig aufwendig gestaltet.

US 4,161,949 und EP-A-0 830 874 beschreiben jeweils Konnektionssysteme, bei denen ein Durchstoßkörper in einem ersten Element zum Durchstoßen einer Membran in einem zweiten Element dient.

In der DE-OS 32 10 148 wird ein gattungsgemäßes Konnektorelement offenbart, das einen Durchstoßkörper aufweist, der relativ zu einem als Elastomermembran ausgeführten Absperrelement bewegbar ist und diese bei der Konnektion öffnet. Der Durchstoßkörper weist auf seiner Öffnungsseite einen männlichen Luer-Konus auf, der bei der Konnektion mit einem entsprechenden weiblichen Konus zusammenwirkt. Die Elastomermembran erstreckt sich über den Bereich zur Führung eines durchströmenden Mediums und verschließt diesen im dekonnektierten Zustand. Die Membran liegt an einer Stufe des Konnektorelementes an und wird dort mittels eines Paßstückes angepreßt. Einem derartigen Konnektorelement ist es nachteilig, daß dieses aus mehreren unterschiedlichen Materialien aufgebaut ist und daß die Membran nachträglich eingeführt und flüssigkeitsdicht fixiert werden muß.

Es ist die Aufgabe der vorliegenden Erfindung, einen gattungsgemäßen Konnektor dahingehend weiterzubilden, daß dieser einfach herstellbar ist und eine zuverlässige Konnektion sicherstellt.

Diese Aufgabe wird mit einem Konnektorelement mit den Merkmalen des Anspruches 1 und einem Konnektionssystem mit den Merkmalen des Anspruches 18 gelöst. Die Erfindung ermöglicht eine verhältnismäßig einfache Herstellung der Konnektorelemente, da das Absperrelement integraler Bestandteil des Konnektorelementes ist und eine nachträgliche Positionierung und Fixierung nicht nötig ist. Ein nachträgliches Aufbringen von Membranen ist somit nicht erforderlich. Dadurch vereinfacht sich nicht nur die Herstellung der Konnektoren, sondern aufgrund der Möglichkeit, die Konnektorelemente und Absperrelemente in einem Arbeitsschritt herzustellen, wird zudem eine besonders dichte Anordnung erreicht. Eine nachträgliche Einschweißung beispielsweise von Folienmembranen oder das nachträgliche Anschweißen oder Anordnen von membranbestückten Einzelbauteilen und eine entsprechend aufwendige Überprüfung können entsprechend entfallen.

Bevorzugte Ausführungsformen sind Gegenstand von Unteransprüchen.

Gemäß einer bevorzugten Ausgestaltung der vorliegenden Erfindung ist das Absperrelement als Spritzgußmembran ausgeführt. Die Spritzgußmembran kann im gleichen Arbeitsgang mit dem Konnektorelement hergestellt werden, wodurch ein nachträgliches Aufbringen von Absperrelementen beliebiger Form überflüssig wird. Insbesondere ergibt sich dadurch eine besonders dichte und totraumfreie Ausgestaltung des erfindungsgemäßen Konnektorelementes. Dabei wird nicht nur ein Auslaufschutz bzw. ein Schutz vor Bakterien sichergestellt, sondern auch eine dauerhafte Dichtigkeit bis zu mehreren Bar Differenzdruck.

In weiterer Ausgestaltung der vorliegenden Erfindung ist vorgesehen, daß der Bereich zur Führung des Mediums den Bereich zur Aufnahme des Durchstoßkörpers umfaßt

Besonders vorteilhaft ist es, wenn der Bereich zur Führung des Mediums einen Rohrstutzen umfaßt.

In weiterer Ausgestaltung der vorliegenden Erfindung ist vorgesehen, daß das Konnektorelement ein Gehäuse aufweist und der Rohrstutzen in dem Gehäuse aufgenommen ist. Dabei kann erfindungsgemäß der Rohrstutzen durch das Absperrelement, beispielsweise eine Membran, verschlossen sein, wodurch die Dichtheit und Sterilität der angeschlossenen Schläuche oder Behälter sichergestellt wird. Vorteilhaft ist es, wenn der Durchstoßkörper in dem Rohrstutzen angeordnet ist. Erfindungsgemäß erfolgt die Anordnung derart, daß bei der Konnektion eine Durchtrennung bzw. Öffnung des Absperrelementes durch den Durchstoßkörper erfolgt. Das Gehäuse kann dazu dienen, den Rohrstutzen sowie das Absperrelement gegen unerwünschtes Berühren und Einbringen von Verunreinigungen zu schützen. Dabei kann der Gehäusedurchmesser derart ausgelegt werden, daß eine versehentliche Berührung des Rohrstutzens bzw. der Bereiche, die nach der Konnektion mit Flüssigkeit oder Gas beaufschlagt werden, nicht möglich ist.

Besonders vorteilhaft ist es, wenn ein Durchstoßkörper vorgesehen ist, der in den Rohrstutzen einführbar und dort mittels einer Arretierung fixierbar ist.

Dabei kann die Arretierung eine am Außenumfang des Durchstoßkörpers umlaufende Nut und einen sich am Innenumfang des Rohrstutzens erstreckenden Vorsprung umfassen. Bei oder nach der Herstellung bzw. vor der Benutzung des Konnektors kann der Durchstoßkörper entsprechend in den Rohrstutzen eingeführt werden und durch die Arretierung in der gewünschten Position gehalten werden.

Gemäß einer bevorzugten Ausgestaltung der vorliegenden Erfindung ist vorgesehen, daß der Durchstoßkörper derart ausgeführt ist, daß dieser durch einen Rohrstutzen eines mit dem Konnektorelement zu verbindenden Konnektorgegenstückes aus der Arretierung lösbar ist. Dadurch wird erreicht, daß erst während der Konnektion der Durchstoßkörper aus seiner Arretierung gelöst wird und mittels eines Rohrstutzens derart bewegt wird, daß die Durchtrennung bzw. Öffnung des Absperrelementes erfolgt. Dabei ist die Arretierung vorteilhaft derart ausgelegt, daß zunächst das Absperrelement des Konnektorelementes durchtrennt wird, bevor der entsprechende Rohrstutzen den Durchstoßkörper aus der Arretierung löst und anschließend das zweite Absperrelement bzw. die zweite Membran durchtrennt.

Das Lösen des Durchstoßkörpers aus der Arretierung kann dadurch erfolgen, daß dieser einen am Außenumfang angeordneten Vorsprung aufweist, der mit dem Rohrstutzen eines mit dem Konnektorelement zu verbindenden Konnektorgegenstückes verbindbar ist.

In weiterer Ausgestaltung der vorliegenden Erfindung ist vorgesehen, daß in dem Gehäuse und dem Rohrstutzen des Konnektorelementes das Gehäuse und der Rohrstutzen eines mit dem Konnektorelement zu verbindenden Konnektorgegenstückes wenigstens teilweise aufnehmbar sind. Dies wird durch eine entsprechende Ausgestaltung der Innen- bzw. Außendurchmesser der aneinandergrenzenden Bauteile erreicht.

Besonders vorteilhaft ist es, wenn der Rohrstutzen in seinem Endbereich abgeschrägt ist. Dadurch wird das Einschieben eines der Rohrstutzen in den diesen aufnehmenden Rohrstutzen erleichtert, wobei vorteilhaft eine Aufweitung des aufnehmenden Rohrstutzens erfolgt, was eine besonders dichte Verbindung beider Rohrstutzen sicherstellt. Die Konnektion und erforderlichenfalls die Dekonnektion kann maschinell oder auch von Hand ausgeführt werden. Vorteilhaft ist es, wenn die Dekonnektion konstruktiv bedingt mit einem höheren Kraftbedarf verbunden ist als die Konnektion. Bei Bedarf lassen sich konstruktive Elemente, beispielsweise spritzgußtechnisch, integrieren, die eine Dekonnektion unmöglich machen.

Vorteilhaft ist es, wenn der Durchstoßkörper eine im wesentlichen zylindrische Form aufweist und an seinen Enden spitz zuläuft. Dadurch wird verhältnismäßig einfache und wenig Kraft beanspruchende Öffnung der Absperrelemente bzw. Membranen ermöglicht. Ebenso ist es möglich, den Durchstoßkörper gekerbt oder gelocht auszuführen oder in einer Sternprofilform vorzusehen.

In weiterer Ausgestaltung der vorliegenden Erfindung ist vorgesehen, daß das Konnektorelement sowie der Durchstoßkörper als Spritzgußteile ausgeführt sind. Die Herstellung läßt sich kostengünstig in Vielfach-Spritzgießwerkzeugen durchführen. Dies ermöglicht eine einfache Herstellung der Teile in vielfältigen möglichen Ausführungsformen.

Besonders vorteilhaft ist es, wenn das Konnektorelement mit Absperrelement sowie der Durchstoßkörper Polypropylen aufweisen.

Gemäß einer bevorzugten Ausgestaltung der vorliegenden Erfindung weisen das Gehäuse sowie der Rohrstutzen des Konnektorelementes zylindrische Außen- und Innenflächen auf. Dies hat den Vorteil, daß weder bei der Herstellung noch bei der Anwendung des Konnektorsystems Drehpositionierungen erforderlich sind. Vielmehr kann die Konnektion durch einfaches Einstecken eines des Konnektorelementes in das Konnektorgegenstück erfolgen. Bei entsprechender Ausgestaltung des Gehäuses des Konnektorelementes und Konnektorgegenstückes kann erreicht werden, daß sich ein besonders guter Berührschutz ergibt. Typische Werte für die Gehäuseinnendurchmesser sind 8,6 mm beim aufnehmenden und 7,4 mm beim einzuführenden Teil, was einen guten Berührschutz auch gegen das Eindringen dünner Fingerkuppen gewährleistet.

Besonders vorteilhaft ist es, wenn die Innen- oder Außenflächen der Rohrstutzen abgeschrägt oder konisch ausgeführt sind. Hierdurch ergibt sich der Vorteil, daß bereits eine dichte Verbindung zwischen zu verbindendem Konnektorelement und -gegenstück hergestellt wird, bevor die Absperrelemente durchstoßen werden.

Die Rohrstutzen können in ihrem Endbereich auf der Innen- oder Außenfläche einen umlaufenden Vorsprung aufweisen, mittels dessen eine dichtende Verbindung mit einem einzuführenden oder aufnehmenden Rohrstutzen eines Konnektorgegenstückes herstellbar ist. Auch hieraus ergibt sich der Vorteil, daß eine Abdichtung gegenüber der Umgebungsatmosphäre erfolgt, bevor es zu einem Durchstoßen der Absperrelemente bzw. der Membranen kommt. Entsprechend gefährden auch Über- und Unterdrücke zwischen den flüssigkeits- und gasgefüllten Zonen des Konnektorelementes bzw. des Konnektorgegenstückes relativ zur Umgebung und Druckunterschiede zwischen Konnektorelement und Konnektorgegenstück, die je nach Dimensionierung der Anordnung bis zu mehreren bar betragen, nicht die Sterilität und die Dichtigkeit vor, während und nach der Konnektion, da stets zuerst eine dichte Verbindung zwischen Konnektorelement und Konnektorgegenstück hergestellt wird, und erst im nächsten Schritt die Verschlußmembranen durchstoßen werden.

Bei der vorliegenden Erfindung ist weiterhin vorgesehen, daß das Absperrelement sowie der Bereich zur Aufnahme des Durchstoßkörpers derart angeordnet sind, daß der Durchstoßkörper von der Konnektionsseite des Konnektorelementes einführbar ist. Dabei ist unter dem Begriff Konnektionsseite die Seite des Konnektorelementes zu verstehen, auf der ein zu verbindendes Konnektorgegenstück eingeführt bzw. aufgesteckt werden soll. Eine derartige Anordnung ist insbesondere dann von Vorteil, wenn das erfindungsgemäße Konnektorelement integraler Bestandteil beispielsweise eines Spritzgußbauteils ist, d.h. wenn das Konnektorelement zusammen mit diesem Bauteil hergestellt wird. Ein derartiges Bauteil kann z.B. ein kassettenartiges Bauteil für die Fluidführung und/oder -behandlung sein. Um Konnektorausgänge zu realisieren, die in verschiedenen und beliebigen Richtungen relativ beispielsweise zu einer Wandung eines kassettenartigen Bauteils verlaufen, ist das Absperrelement bzw. die Membran derart angeordnet, daß der Durchstoßkörper von außen bzw. von der Konnektionsseite einführbar ist.

Weitere Einzelheiten und Vorteile der vorliegenden Erfindung werden anhand eines in der Zeichnung dargestellten Ausführungsbeispiels näher erläutert. Es zeigen:
- Fig. 1:: Eine Schnittdarstellung durch ein erfindungsgemäßes Konnektorelement und ein Konnektorgegenstück in einer Grundstellung vor der Konnektion,
- Fig. 2:: das Konnektorelement und das Konnektorgegenstück gemäß Fig. 1 nach dem Einführen des rechts dargestellten Konnektorgegenstükkes bis zum Anliegen der Membran an dem Durchstoßkörper,
- Fig. 3:: das Konnektorelement und das Konnektorgegenstück gemäß Fig. 1 nach dem Öffnen der Membran des rechts dargestellten Konnektorgegenstückes und
- Fig. 4:: das Konnektorelement und das Konnektorgegenstück gemäß Fig. 1 nach dem Durchtrennen beider Membranen durch den Durchstoßkörper.

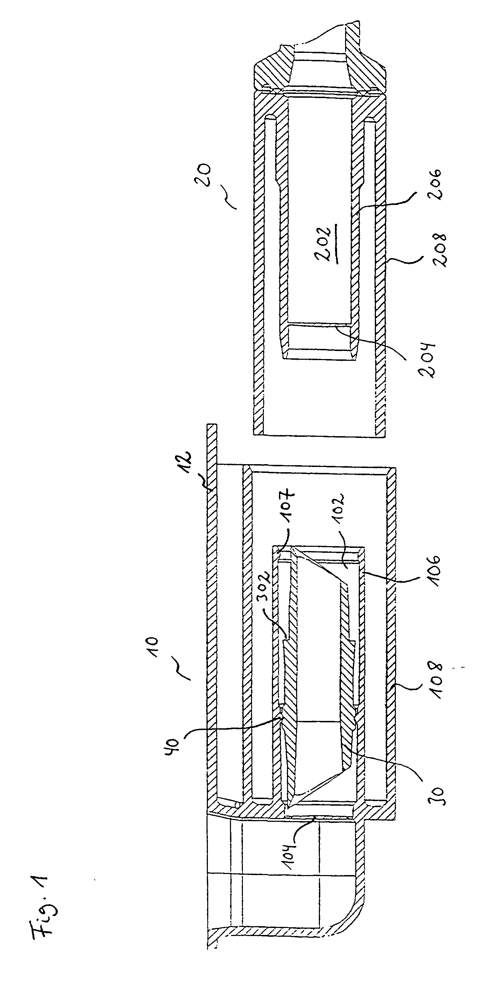

Fig. 1 zeigt ein Konnektorelement 10 und ein Konnektorgegenstück 20 vor dem Zusammenführen in einer Grundposition. Das Konnektorelement 10 weist das Gehäuse 108 auf, in dem sich der Rohrstutzen 106 erstreckt. An dem sich gemäß Fig. 1 links erstreckenden Ende des Gehäuses 108 befindet sich eine Öffnung beispielsweise zur Aufnahme eines Schlauchendes. Diese Öffnung sowie der durch den Rohrstutzen 106 begrenzte Bereich definieren den Bereich 102 zur Führung eines durchströmenden Mediums, beispielsweise einer Flüssigkeit oder eines Gases. Der Bereich 102 ist durch die als Absperrelement dienende Membran 104 dicht verschlossen.

In dem Rohrstutzen 106 ist der Durchstoßkörper 30 aufgenommen, der mittels der Arretierung 40 in einer gewünschten Position im Rohrstutzen 106 gehalten wird. Die Arretierung 40 besteht gemäß dem vorliegenden Ausführungsbeispiel aus einem sich am Innenumfang des Rohrstutzens 106 erstreckenden Vorsprung, der in einer entsprechenden umlaufenden Nut am Außenumfang des Durchstoßkörpers 30 eingreift. Der Durchstoßkörper weist an seinem Außenumfang einen umlaufenden Vorsprung 302 auf.

Das Konnektorgegenstück 20 weist ein Gehäuse 208 auf, in dem der Rohrstutzen 206 aufgenommen ist. In dem in Fig. 1 rechts dargestellten Teil des Gehäuses 208 kann ein Schlauch oder ein beliebiges Arbeitsmittel angeschlossen werden. Der Rohrstutzen 206 sowie sich der daran anschließende Bereich zur Aufnahme eines Schlauches oder Arbeitsmittels begrenzen den Bereich 202 zur Führung eines durchströmenden Mediums. Dieser ist durch eine als Absperrelement dienende Membran 204 im Bereich des Rohrstutzens 206 dicht verschlossen. Somit ergibt sich auch für das Konnektorgegenstück 20 eine dichte und zuverlässige Absperrung gegen das unerwünschte Eindringen von Verunreinigungen.

In dem gemäß Fig. 1 rechts dargestellten Endbereich des Rohrstutzens 106 befindet sich der umlaufende Vorsprung 107. Dieser dient dazu, während der Konnektion des Konnektorelementes 10 und des Konnektorgegenstückes 20 eine dichte Verbindung zwischen den Rohrstutzen 106, 206 zu bewirken. Daraus ergibt sich der Vorteil, daß vor dem Durchstoßen der Absperrelemente 104, 204 bereits eine Abdichtung gegen die Umgebung erfolgt, wodurch die Sterilität und die Dichtigkeit der Verbindung vor, während und nach der Konnektion sichergestellt wird.

Die Membranen 104, 204 sind als Spritzgußmembranen hermetisch dicht, totraumfrei und monomaterial ausgeführt, d.h., deren Herstellung erfolgt gleichzeitig mit der Herstellung des Konnektorelementes 10 bzw. des Konnektorgegenstückes 20. Daraus ergibt sich eine verhältnismäßig einfache Herstellbarkeit der erfindungsgemäßen Konnektoren und zum anderen eine besonders hohe Zuverlässigkeit der Membranen. Insbesondere ist eine nachträgliche Einschweißung von Folienmembranen oder anderen Absperrelementen und eine entsprechende Überprüfung überflüssig. Vielmehr können die erfindungsgemäßen Spritzgußmembranen in der Startphase der Serienproduktion zu 100 % und später im Stichprobenverfahren durch einfache Druckhalteprüfungen auf Integrität geprüft werden. Diese Prüfung sowie weitere Eigenschaftsprüfungen erfolgen vorteilhaft schon beim Lieferanten der Spritzgußteile bzw. in den Labors der Qualitätskontrolle. Eine Kontrolle am Hauptmontageband bzw. die Überprüfung nachträglich gefügter Membranen wird überflüssig. Darüber hinaus entfällt die Aussonderung von Ausschuß aufgrund Undichtigkeit am Hauptmontageband. Eine typische Stärke der die Absperrelemente 104, 204 bildenden Spritzgußmembranen beträgt 0,2 mm.

Das Konnektorelement 10 ist gemäß dem vorliegenden Ausführungsbeispiel als integraler Bestandteil eines kassettenartigen Bauteils für die Fluidführung und/oder -behandlung ausgeführt und wird zusammen mit diesem hergestellt. Die Wandung 12 des kassettenartigen Bauteils befindet sich unmittelbar oberhalb des Gehäuses 108. Um beliebige Richtungen des Ausgangs des Konnektorelementes 10 relativ zur Kassettenwand 12 zu ermöglichen, ist der Bereich 102 zur Aufnahme des Durchstoßkörpers 30 sowie das Absperrelement 104 derart angeordnet, daß der Durchstoßkörper von der gemäß Fig. 1 rechts dargestellten Konnektionsseite des Konnektorelementes 10 einführbar ist. Dadurch wird es möglich, die Konnektionsrichtung in beliebiger Orientierung relativ zu dem kassettenartigen Bauteil bzw. zu dessen Wandung 12 auszuführen. Aufgrund einer derartigen Ausführung kann der Durchstoßkörper problemlos in den entsprechenden Aufnahmebereich eingebracht werden.

Um einen weiten Anwendungsbereich eines derartigen kassettenartigen Bauteils zu gewährleisten ist es besonders vorteilhaft, den Durchstoßkörper 30 in das kassettenseitige Konnektorelement 10 einzubringen. Dies ist insbesondere dann erforderlich, wenn das mit dem kassettenseitigen Konnektorelement zu verbindende Konnektorelement nicht von einem Disposable, wie z.B. einem Schlauch, herrührt, sondern direkt einen maschinenseitigen Anschluß darstellt, der nicht ständig ausgewechselt wird.

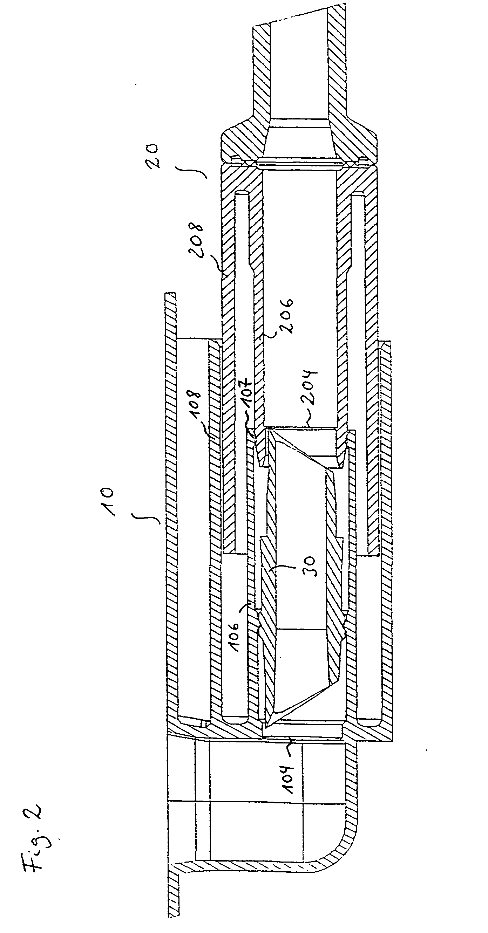

Werden das Konnektorelement 10 und das Konnektorgegenstück 20 ausgehend von der in Fig. 1 dargestellten Position zusammengefügt, erfolgt zunächst ein Einführen des Gehäuses 208 und des Rohrstutzens 206 des Konnektorgegenstückes 20 in das Gehäuse 108 und den Rohrstutzen 106 des Konnektorelementes 10 bis das als Membran ausgeführte Absperrelement 204 am rechten Endbereich des Durchstoßkörpers 30 anliegt, wie dies in Fig. 2 dargestellt ist. Beim Zusammenführen werden das Gehäuse 208 in das Gehäuse 108 und der Rohrstutzen 206 in den Rohrstutzen 106 eingefügt. Die Rohrstutzen 106, 206 sind, um diese Einführung zu erleichtern, in ihren Endbereichen abgeschrägt. Neben der in Fig. 2 gezeigten Anordnung ist es möglich, daß das Gehäuse 108 und der Rohrstutzen 106 in die entsprechenden Teile des Konnektorgegenstückes 20 aufgenommen werden.

Während des Zusammenfügens des Konnektorelementes 10 und des Konnektorgegenstückes 20 gemäß Fig. 2 kommt es zu einer Verbindung des umlaufenden Vorsprungs 107 des Rohrstutzens 106 mit der Außenfläche des eingeführten Rohrstutzens 206 des Konnektorgegenstückes 20. Hierdurch wird sichergestellt, daß bereits eine Abdichtung gegen die Umgebungsatmosphäre erfolgt, bevor die Absperrelemente 104, 204 durchstoßen werden.

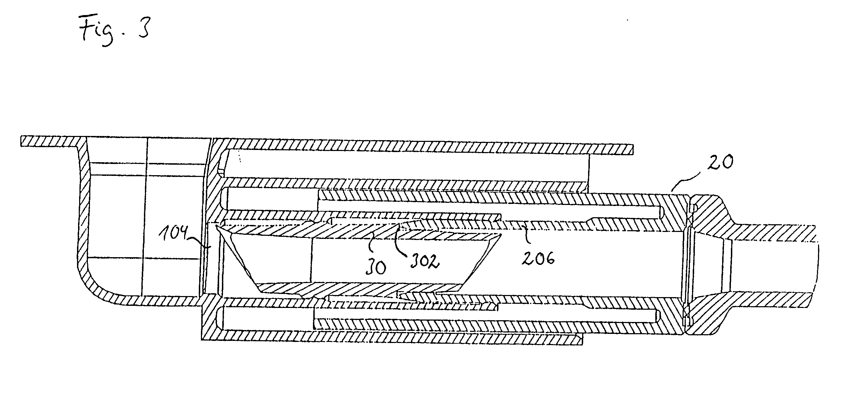

Werden Konnektorelement und Konnektorgegenstück ausgehend vom Zustand der Fig. 2 weiter zusammengeführt, ergibt sich der in Fig. 3 dargestellte Zustand. Hier hat bereits die rechte, spitz zulaufende Kante des Durchstoßkörpers 30 das Absperrelement des Konnektorgegenstückes 20 durchtrennt. Ferner liegt der Endbereich des Rohrstutzens 206 am Vorsprung 302 des Durchstoßkörpers 30 an. In diesem Stadium ist bereits eines der als Membranen ausgeführten Absperrelemente durchtrennt, während das andere Absperrelement 104 noch verschlossen ist. Dies ist darauf zurückzuführen, daß der Durchstoßkörper 30 bis zu dem in Fig. 3 dargestellten Zustand in der arretierten Position gehalten wird.

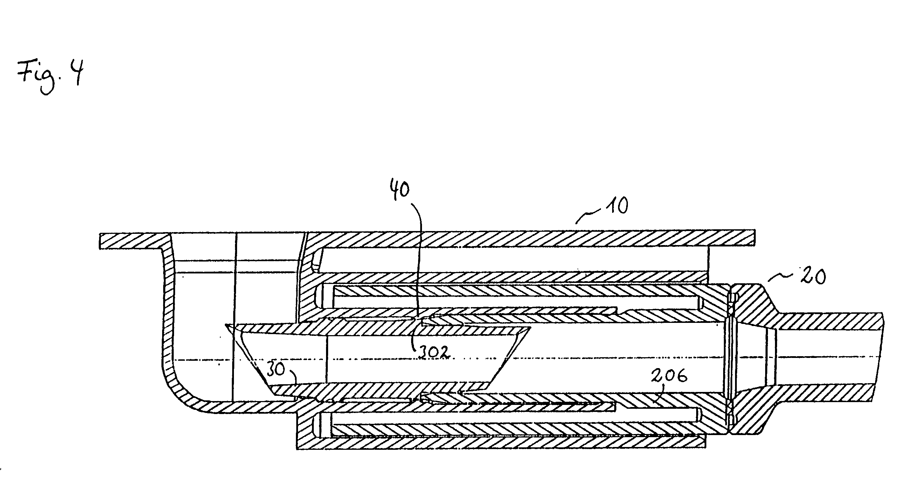

Ein weiteres Verschieben bzw. Zusammenführen des Konnektorelementes 10 und des Konnektorgegenstückes 20 führt schließlich zu der in Fig. 4 dargestellten Anordnung. Der Kontakt des vorderen Endes des Rohrstutzens 206 des Konnektorgegenstückes 20 mit dem Vorsprung 302 des Durchstoßkörpers 30 führt dazu, daß der Durchstoßkörper 30 aus der Arretierung 40 gelöst wird und mit seinem links dargestellten spitzen Ende das als Membran ausgeführte Absperrelement durchtrennt. Damit liegt ein zuverlässig abgedichteter und steriler Durchlaß zwischen Konnektorelement 10 und Konnektorgegenstück 20 vor.

Neben der in Fig. 1 bis 4 dargestellten Ausführungsform ist es ebenfalls möglich, daß zunächst das Absperrelement 104 des Konnektorelementes 10 durchtrennt wird und anschließend das Absperrelement 204 des Konnektorgegenstückes 20. Dazu wäre erforderlich, daß der Durchstoßkörper 30 nach dem Durchtrennen des Absperrelementes 104 an einem Anschlag anliegt, wodurch eine Relativbewegung zwischen Durchstoßkörper 30 und dem Absperrelement 204 des Konnektorgegenstückes 20 erfolgen kann.

Der Innendurchmesser der Gehäuse 108, 208 kann vorteilhaft derart gewählt werden, daß sich ein wirksamer Berührschutz dadurch ergibt, daß ein Eindringen mit Fingern unmöglich wird. Vorteilhaft weist das aufnehmende Teil (Konnektorelement oder -gegenstück) einen Innendurchmesser von 8,6 mm und das einzuführende Teil (Konnektorelement oder -gegenstück) einen Innendurchmesser von 7,4 mm auf. Typische Werte für den Außendurchmesser und die Einstecktiefe des einzuführenden Teiles (Konnektorelement oder -gegenstück) sind 8,4 mm bzw. minimal 22 mm.

Das erfindungsgemäße Konnektorelement gewährleistet Dichtheit vor, während und nach der Konnektion. Für den sicheren Konnektionsvorgang spielt es darüber hinaus keine Rolle, ob die Konnektoren flüssigkeits- oder gasgefüllt sind. Nachträgliche Konnektionen während des Einsatzes sind also möglich. Dabei spielt die Flußrichtung keine Rolle.

## Patentansprüche

1. Konnektorelement (10), insbesondere zum Verbinden von Schläuchen, Kanülen und Kathetern, mit
- einem Bereich (102) zur Führung eines durchströmenden Mediums,
- einem Absperrelement (104), durch das der Bereich (102) zur Führung des Mediums verschließbar ist, und
- einem Durchstoßkörper (30), der derart angeordnet ist, daß er relativ zu dem Absperrelement (104) bewegbar ist und das Absperrelement (104) bei der Konnektion mit einem Konnektorgegenstück (20) öffnet,
**dadurch gekennzeichnet, daß**
- das Absperrelement (104) als monomaterialer Bestandteil des Konnektorelementes (10) ausgeführt ist, und
- das Absperrelement (104) sowie der Bereich (102) zur Aufnahme des Durchstoßkörpers (30) derart angeordnet sind, daß der Durchstoßkörper (30) von der Konnektionsseite des Konnektorelementes (10) einführbar ist.

2. Konnektorelement (10) nach Anspruch 1, **dadurch gekennzeichnet, daß** das Absperrelement (104) als Spritzgußmembran ausgeführt ist.

3. Konnektorelement (10) nach Anspruch 1 oder 2, **dadurch gekennzeichnet, daß** der Bereich (102) zur Führung des Mediums den Durchstoßkörper (30) aufnimmt.

4. Konnektorelement (10) nach einem oder mehreren der Ansprüche 1 bis 3, **dadurch gekennzeichnet, daß** der Bereich (102) zur Führung des Mediums einen Rohrstutzen (106) umfaßt.

5. Konnektorelement (10) nach Anspruch 4, **dadurch gekennzeichnet, daß** das Konnektorelement (10) ein Gehäuse (108) aufweist und der Rohrstutzen (106) in dem Gehäuse (108) aufgenommen ist.

6. Konnektorelement (10) nach Anspruch 4 oder 5, **dadurch gekennzeichnet, daß** ein Durchstoßkörper (30) vorgesehen ist, der in den Rohrstutzen (106) einführbar und dort mittels einer Arretierung (40) fixierbar ist.

7. Konnektorelement (10) nach Anspruch 6, **dadurch gekennzeichnet, daß** die Arretierung (40) eine am Außenumfang des Durchstoßkörpers (30) umlaufende Nut und einen sich am Innenumfang des Rohrstutzens (106) erstreckenden Vorsprung umfaßt.

8. Konnektorelement (10) nach Anspruch 6 oder 7, **dadurch gekennzeichnet, daß** der Durchstoßkörper (30) derart ausgeführt ist, daß dieser durch einen Rohrstutzen (206) eines mit dem Konnektorelement (10) zu verbindenden Konnektorgegenstückes (20) aus der Arretierung (40) lösbar ist.

9. Konnektorelement (10) nach Anspruch 8, **dadurch gekennzeichnet, daß** der Durchstoßkörper (30) einen am Außenumfang angeordneten Vorsprung (302) aufweist, der mit dem Rohrstutzen (206) eines mit dem Konnektorelement (10) zu verbindenden Konnektorgegenstückes (20) verbindbar ist.

10. Konnektorelement (10) nach einem oder mehreren der Ansprüche 5 bis 9, **dadurch gekennzeichnet, daß** in dem Gehäuse (108) und dem Rohrstutzen (106) des Konnektorelementes (10) das Gehäuse (208) und der Rohrstutzen (206) eines mit dem Konnektorelement (10) zu verbindenden Konnektorgegenstückes (20) wenigstens teilweise aufnehmbar sind.

11. Konnektorelement (10) nach einem oder mehreren der Ansprüche 4 bis 10, **dadurch gekennzeichnet, daß** der Rohrstutzen (106) in seinem Endbereich abgeschrägt ist.

12. Konnektorelement (10) nach einem oder mehreren der Ansprüche 1 bis 11, **dadurch gekennzeichnet, daß** der Durchstoßkörper (30) eine im wesentlichen zylindrische Form aufweist und an seinen Enden spitz zuläuft.

13. Konnektorelement (10) nach einem oder mehreren der Ansprüche 1 bis 12, **dadurch gekennzeichnet, daß** das Konnektorelement (10) sowie der Durchstoßkörper (30) als Spritzgußteile ausgeführt sind.

14. Konnektorelement (10) nach einem oder mehreren der Ansprüche 1 bis 13, **dadurch gekennzeichnet, daß** das Konnektorelement (10) mit Absperrelement (104) sowie der Durchstoßkörper (30) Polypropylen aufweisen.

15. Konnektorelement (10) nach einem oder mehreren der Ansprüche 4 bis 14, **dadurch gekennzeichnet, daß** das Gehäuse (108) sowie der Rohrstutzen (106) zylindrische Außen- und Innenflächen aufweisen.

16. Konnektorelement (10) nach einem oder mehreren der Ansprüche 4 bis 15, **dadurch gekennzeichnet, daß** die Innen- oder Außenflächen der Rohrstutzen (106) abgeschrägt oder konisch ausgeführt sind.

17. Konnektorelement (10) nach einem oder mehreren der Ansprüche 4 bis 16, **dadurch gekennzeichnet, daß** die Rohrstutzen (106) in ihrem Endbereich auf der Innen- oder Außenfläche einen umlaufenden Vorsprung (107) aufweisen, mittels dessen eine dichtende Verbindung mit einem einzuführenden (206) oder aufnehmenden Rohrstutzen eines Konnektorgegenstückes (20) herstellbar ist.

18. Konnektorsystem, insbesondere zum Verbinden von Schläuchen, Kanülen und Kathetern, mit einem Konnektorelement (10) gemäß einem der Ansprüche 1 bis 17 und einem mit dem Konnektorelement (10) zur Konnektion verbindbaren Konnektorgegenstück (20).

## Claims

1. Connector element (10), in particular for the joining of tubes, cannulae and catheters, with
- a section (102) for guiding a through-flowing medium,
- a shut-off element (104) by which the section (102) for guiding the medium can be sealed, and
- a pusher (30) which is arranged such that it is movable relative to the shut-off element (104) and opens the shut-off element (104) upon connection with a connector counterpiece (20),
**characterized in that**
- the shut-off element (104) is designed as a single-material constituent of the connector element (10), and
- the shut-off element and the section (102) for housing the pusher (30) are arranged such that the pusher (30) can be introduced from the connection side of the connector element (10).

2. Connector element (10) according to claim 1, **characterized in that** the shut-off element (104) is designed as an injection moulding membrane.

3. Connector element (10) according to claim 1 or 2, **characterized in that** the section (102) for guiding the medium houses the pusher (30).

4. Connector element (10) according to one or more of claims 1 to 3, **characterized in that** the section (102) for guiding the medium comprises a sleeve (106).

5. Connector element (10) according to claim 4, **characterized in that** the connector element (10) has a housing (108) and the sleeve (106) is housed in the housing (108).

6. Connector element (10) according to claim 4 or 5, **characterized in that** a pusher (30) is provided which can be introduced into the sleeve (106) and fixed there by means of a lock (40).

7. Connector element (10) according to claim 6, **characterized in that** the lock (40) comprises a groove running round the outer periphery of the pusher (30) and a projection extending along the inner periphery of the sleeve (106).

8. Connector element (10) according to claim 6 or 7, **characterized in that** the pusher (30) is designed such that it can be released from the lock (40) by a sleeve (206) of a connector counterpiece (20) to be joined to the connector element (10).

9. Connector element (10) according to claim 8, **characterized in that** the pusher (30) has a projection (302), arranged on the outer periphery, which can be joined to the sleeve (206) of a connector counterpiece (20) to be joined to a connector element (10).

10. Connector element (10) according to one or more of claims 5 to 9, **characterized in that** the housing (208) and the sleeve (206) of a connector counterplece (20) to be joined to the connector element (10) can be at least partly housed in the housing (108) and the sleeve (106) of the connector element (10).

11. Connector element (10) according to one or more of claims 4 to 10, **characterized in that** the sleeve (106) is bevelled in its end-region.

12. Connector element (10) according to one or more of claims 1 to 11, **characterized in that** the pusher (30) has an essentially cylindrical form and is pointed at its ends.

13. Connector element (10) according to one or more of claims 1 to 12, **characterized in that** the connector element (10) and the pusher (30) are designed as injection mouldings.

14. Connector element (10) according to one or more of claims 1 to 13, **characterized In that** the connector element (10) with shut-off element (104) and the pusher (30) have polypropylene.

15. Connector element (10) according to one or more of claims 4 to 14, **characterized in that** the housing (108) and the sleeve (106) have cylindrical outer and inner surfaces.

16. Connector element (10) according to one or more of claims 4 to 15, **characterized in that** the inner or outer surfaces of the sleeve (106) are bevelled or conical.

17. Connector element (10) according to one or more of claims 4 to 16, **characterized in that** in their end-region the sleeves (106) have on the inner or outer surface a peripheral projection (107), by means of which a sealing joint with a sleeve (206) of a connector element (20) to be introduced or housed can be created.

18. Connector system, in particular for the joining of tubes, cannulae and catheters, with a connector element (10) according to one of claims 1 to 17 and a connector counterpiece (20) that can be joined to the connector element (10) for connection.

## Revendications

1. Elément connecteur (10), en particulier pour relier des tuyaux, des canules et des cathéters, avec
- une zone (102) pour le guidage d'un milieu traversant,
- un élément de fermeture (104) par lequel la zone (102) pour le guidage du milieu peut être fermée et
- un corps perforateur (30) disposé de telle manière qu'il est déplaçable relativement à l'élément de fermeture (104) et qu'il ouvre l'élément de fermeture (104) lors de la connexion avec un pendant de connecteur (20),
**caractérisé**
- **en ce que** l'élément de fermeture (104) est réalisé comme composant mono-matériel de l'élément connecteur (10) et
- **en ce que** l'élément de fermeture (104) ainsi que la zone (102) pour la réception du corps perforateur (30) sont disposés de façon que le corps perforateur (30) puisse être inséré depuis le côté connexion de l'élément connecteur (10).

2. Elément connecteur (10) selon la revendication 1, **caractérisé en ce que** l'élément de fermeture (104) est réalisé comme une membrane moulée par injection.

3. Elément connecteur (10) selon la revendication 1 ou 2, **caractérisé en ce que** la zone (102) pour le guidage du milieu reçoit le corps perforateur (30).

4. Elément connecteur (10) selon l'un ou plusieurs des revendications 1 à 3, **caractérisé en ce que** la zone (102) pour le guidage du milieu comprend une tubulure (106).

5. Elément connecteur (10) selon la revendication 4, **caractérisé en ce que** l'élément connecteur (10) présente un boîtier (108), et que la tubulure (106) est reçue dans le boîtier (108).

6. Elément connecteur (10) selon la revendication 4 ou 5, **caractérisé en ce qu'**un corps perforateur (30) est prévu, qui peut être inséré dans la tubulure (106) et y être fixé au moyen d'un arrêtage (40).

7. Elément connecteur (10) selon la revendication 6, **caractérisé en ce que** l'arrêtage (40) comprend une rainure s'étendant à la périphérie extérieure du corps perforateur (30) et une saillie s'étendant à la périphérie intérieure de la tubulure (106).

8. Elément connecteur (10) selon la revendication 6 ou 7, **caractérisé en ce que** le corps perforateur (30) est réalisé de telle sorte que celui-ci peut être relâché de l'arrêtage (40), par un tubulure (206) d'un pendant de connecteur (20) à relier à l'élément connecteur (10).

9. Elément connecteur (10) selon la revendication 8, **caractérisé en ce que** le corps perforateur (30) présente une saillie (302) disposée au pourtour extérieur qui peut être reliée à la tubulure (206) d'un pendant de connecteur (20) à relier à l'élément connecteur (10).

10. Elément connecteur (10) selon l'une ou plusieurs des revendications 5 à 9, **caractérisé en ce que** dans le boîtier (108) et la tubulure (106) de l'élément connecteur (10), le boîtier (208) et la tubulure (206) d'un pendant de connecteur (20) à relier à l'élément connecteur (10) peuvent être reçus au moins partiellement.

11. Elément connecteur (10) selon l'une ou plusieurs des revendications 4 à 10, **caractérisé en ce que** la tubulure (106) est chanfreinée dans sa zone d'extrémité.

12. Elément connecteur (10) selon l'une ou plusieurs des revendications 1 à 11, **caractérisé en ce que** le corps perforateur (30) présente une forme sensiblement cylindrique et se termine en pointe à ses extrémités.

13. Elément connecteur (10) selon l'une ou plusieurs des revendications 1 à 12, **caractérisé en ce que** l'élément connecteur (10) ainsi que le corps perforateur (30) sont réalisés comme des parties moulées par injection.

14. Elément connecteur (10) selon l'une ou plusieurs des revendications 1 à 13, **caractérisé en ce que** l'élément connecteur (10) avec l'élément de fermeture (104) ainsi que le corps perforateur (30) présentent du prolypopylène.

15. Elément connecteur (10) selon l'une ou plusieurs des revendications 4 à 14, **caractérisé en ce que** le boîtier (108) ainsi que la tubulure (106) présentent des faces extérieures et intérieures cylindriques.

16. Elément connecteur (10) selon l'une ou plusieurs des revendications 4 à 15, **caractérisé en ce que** les faces extérieures ou intérieures des tubulures (106) sont chanfreinées ou réalisées d'une manière conique.

17. Elément connecteur (10) selon l'une ou plusieurs des revendications 4 à 16, **caractérisé en ce que** les tubulures (106) présentent dans leur zone d'extrémité, sur la face intérieure ou extérieure, une saillie (107) s'étendant tout autour au moyen de laquelle peut être réalisée une liaison étanche avec une tubulure à insérer (206) ou à recevoir d'un pendant de connecteur (20).

18. Système de connecteur, en particulier pour relier des tuyaux, des canules et des cathéters, avec un élément connecteur (10) selon l'une des revendications 1 à 17 et avec un pendant de connecteur (20) pouvant être relié à l'élément connecteur (10) en vue de la connexion.
